# EUROPEAN PATENT SPECIFICATION

(11) **EP 2 471 775 B1**
(45) Date of publication and mention of the grant of the patent: **23.01.2013**
(21) Application number: 12162660.0
(22) Date of filing: 18.03.2009
(51) Int. Cl.: C07D 211/56, B01J 23/44, C07B 57/00, C07B 61/00, C07D 213/75

(54) **Manufacturing method for a piperidine-3-ylcarbamate compound and optical resolution method therefor**
Herstellungsverfahren für eine Piperidin-3-ylcarbamat Verbindung und Verfahren zur optischen Auflösung dafür
Procédé de fabrication d'un composé de pipéridine-3-ylcarbamate et procédé de résolution optique correspondant

(30) Priority: 26.03.2008 JP 2008080075; 19.05.2008 JP 2008130407
(43) Date of publication of application: 04.07.2012
(62) Divisional of application: 09726287.7
(73) Proprietor: SUMITOMO CHEMICAL CO., LTD., Tokyo 104-8260 (JP)
(72) Inventor: Watanabe, Yosuke, Toyonaka-shi Osaka 561-0021 (JP); Yasuoka, Junichi, Kobe-shi Hyogo 658-0073 (JP); Ikemoto, Tetsuya, Toyonaka-shi Osaka 561-0874 (JP)
(74) Representative: Wallace, Sheila Jane

(56) References cited:
- EP-A1- 2 248 803
- WO-A1-00/71534
- WO-A1-2007/075630

## Description

### TECHNICAL FIELD

The present invention relates to a manufacturing method for a piperidin-3-ylcarbamate compound and an optical resolution method therefor.

### BACKGROUND ART

As a manufacturing method for a piperidin-3-ylcarbamate compound, there is known a method of bringing a corresponding pyridin-3-ylcarbamate compound and hydrogen into contact in the presence of a rhodium catalyst (See Patent document 1). However, since a rhodium catalyst used in such a method is expensive, development of a further more economical manufacturing method for a piperidin-3-ylcarbamate compound has been demanded.

Further, as a manufacturing method for an optically active 3-aminopiperidine, there is known, for example, a method by optical resolution of 3-aminopiperidine (See Patent document 2). However, such a method has not been industrially satisfactory in terms of the optical purity of the obtained 3-aminopiperidine.

Another known method for the production of optically active pyridin-3-ylcarbamate compounds takes chiral amino acid starting materials and converts them to desired products(See Patent document 3).

[Patent document 1] United States Patent Application Laid-open No. 2005/0159423 Specification
[Patent document 2] International Application Publication No. 2007/75630
[Patent document 3] International Application Publication No. 2000/71534

### DISCLOSURE OF THE INVENTION

Under such circumstances, the present inventors have made eager studies on a manufacturing method for a piperidin-3-ylcarbamate compound, and have found out that a piperidin-3-ylcarbamate compound can be obtained by bringing a pyridin-3-ylcarbamate compound and hydrogen into contact in the presence of a palladium catalyst which is less expensive. Further, the present inventors have found out that a piperidin-3-ylcarbamate compound can be obtained with a good yield when the pH value in the reaction system is adjusted to be within a range from 1 to 7 by using a carboxylic acid compound or phosphoric acid.

Furthermore, the present inventors have made eager studies on a method of obtaining 3-aminopiperidine having a higher optical purity, and have found out that a piperidin-3-ylcarbamate compound or a salt thereof having a high optical purity can be obtained when ethyl piperidin-3-ylcarbamate or t-butyl piperidin-3-ylcarbamate having a structure such that the amino group at 3-position of 3-aminopiperidine is protected by an ethoxycarbonyl group or a t-butoxycarbonyl group is subjected to optical resolution with use of an optically active mandelic acid.

That is, the present invention provides the following inventions disclosed in [1] to [8].
[1] An optical resolution method of a piperidin-3-ylcarbamate compound characterized in that an RS mixture of a piperidin-3-ylcarbamate compound represented by the formula (1) : 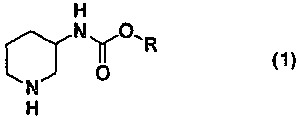 (in the formula, R has an ethyl group or a t-butyl group) and optically active mandelic acid are brought into contact.
[2] The optical resolution method according to [21], wherein the optically active mandelic acid is R-mandelic acid.
[3] A manufacturing method for an optically active piperidin-3-ylcarbamate compound represented by the formula (2) : 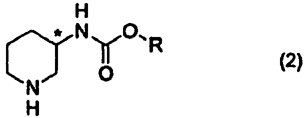 (in the formula, R represents an ethyl group or a t-butyl group, and * represents that the carbon atom is an optically active center) or a salt thereof, comprising the optical resolution method according to [1] to crystallize a diastereomer salt of the optically active piperidin-3-ylcarbamate compound and the optically active mandelic acid, and then allowing an acid or an alkali to react with the diastereomer salt.
[4] The manufacturing method according to [3], wherein the optically active mandelic acid is R-mandelic acid, and the obtained optically active piperidin-3-ylcarbamate compound represented by the formula (2) is an R form.
[5] A diastereomer salt of optically active ethyl piperidin-3-ylcarbamate and optically active mandelic acid.
[6] A diastereomer salt of optically active t-butyl piperidin-3-ylcarbamate and optically active mandelic acid.
[7] A diastereomer salt of ethyl (R)-piperidin-3-ylcarbamate and R-mandelic acid.
[8] A diastereomer salt of t-butyl (R)-piperidin-3-ylcarbamate and R-mandelic acid.

According to the present invention, a piperidin-3-ylcarbamate compound can be manufactured at a lower cost, thereby being industrially advantageous. Further, according to the present invention, a piperidin-3-ylcarbamate compound or a salt thereof having a high optical purity can be obtained. The obtained optically active piperidin-3-ylcarbamate compound is subjected to deprotection to give 3-aminopiperidine having a high optical purity, thus it is industrially advantageous.

### MODES FOR CARRYING OUT THE INVENTION

Hereafter, the present invention will be described in detail.

The starting piperidine-3-ylcarbamate compound (1) may be obtained from the pyridin-3-ylcarbamate compound shown by the formula (3) (hereafter abbreviated as pyridin-3-ylcarbamate compound (3)) will be described.

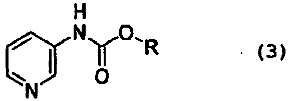

In the formula, R represents ethyl, or t-butyl

Examples of the pyridin-3-ylcarbamate compound (3) include ethyl pyridin-3-ylcarbamate and t-butyl pyridin-3-ylcarbamate.

The pyridin-3-ylcarbamate compound (3) can be manufactured typically by a method such as a conversion of the amino group at 3-position of 3-aminopyridine to carbamate by a conventional method or allowing nicotinic acid amide to undergo Hofmann rearrangement in the presence of an alcohol. It is preferable to lead the amino group at 3-position of 3-aminopyridine to carbamate.

The aforesaid carbamation (carbamate formation) is carried out, for example, by using an alkyl halocarbonate such as alkyl chlorocarbonate. Here, the ester moiety in the alkyl halocarbonate corresponds to R in the formula (3). For example, when R is a t-butyl group, it is possible to carbamate with use of a t-butyl halocarbonate; however, it is general to performing a carbamation with use of a di-t-butyl dicarbonate. Of course, when R is another alkyl group within the scope of the invention, one may performing a carbamation with use of a corresponding dialkyl dicarbonate. In the following, the alkyl halocarbonate and the dialkyl dicarbonate may be generally referred to as a "carbamation agent".

Such carbamation is typically carried out in the presence of a solvent. Examples of the solvent include water; an ether solvent such as tetrahydrofuran and t-butyl methyl ether; a nitrile solvent such as acetonitrile; an ester solvent such as ethyl acetate; an aromatic solvent such as toluene; and an alcohol solvent such as methanol, 1-butanol, 2-propanol and 2-methyl-2-propanol. These solvents may be used either alone or as a combination of two or more kinds. The amount of use of the solvent is typically 1 to 50 L, preferably 2 to 20 L, relative to 1 kg of 3-aminopyridine.

Further, such carbamation may be carried out in the presence of a base. Examples of the base include hydrogencarbonate salt such as sodium hydrogencarbonate and potassium hydrogencarbonate; carbonate salt such as sodium carbonate and potassium carbonate; metal hydroxide such as sodium hydroxide and potassium hydroxide; and an organic base such as triethylamine, diisobutylethylamine and pyridine. These bases may be used either alone or as a combination of two or more kinds. The amount of use of the base is typically 1 to 5 times by mol, preferably 1 to 2 times by mol, relative to 3-aminopyridine.

The reaction temperature for carbamation is typically -20°C to 50°C, preferably -10°C to 30°C. The reaction time is typically 15 minutes to 12 hours, preferably 30 minutes to 5 hours, though it may depend on the reaction temperature, the amount of use of the reaction reagents, and the like. Progress of the reaction can be confirmed by ordinary means such as gas chromatography or high-speed liquid chromatography.

The order of mixing in the carbamation is not particularly limited. For example, a mode in which a base and a carbamation agent are added simultaneously in parallel to 3-aminopyridine or a mode in which a carbamation agent is added into a mixture of 3-aminopyridine and a base.

The mixture after the reaction is finished contains a pyridin-3-ylcarbamate compound (3), and this may be subjected to contact with hydrogen. Typically, however, the mixture is subjected to contact with hydrogen after being subjected to an ordinary post-treatment such as filtration, extraction, or water-washing. Of course, the mixture after the aforesaid post-treatment may be subjected to contact with hydrogen after being subjected to an ordinary isolation process such as distillation or crystallization, or may be subjected to contact with hydrogen after being subjected to an ordinary purification process such as recrystallization; extraction purification; distillation; adsorption process with activated carbon, silica, alumina, or the like; a chromatography method such as silica gel column chromatography; or the like.

Next, the contact of pyridin-3-ylcarbamate compound (3) and hydrogen carried out in the presence of a palladium catalyst (which may hereafter be referred to as the reduction reaction) will be described.

The palladium catalyst may be a compound containing palladium; however, palladium carbon is typically used. The palladium carbon is preferably one in which palladium is carried at 1 to 20% of the total weight on carbon. The palladium carbon may contain water or may be a dry product. The amount of use of the palladium catalyst is an amount within a range such that palladium atoms are contained typically at 0.01 to 10 wt%, preferably 0.1 to 5 wt%, relative to the pyridin-3-ylcarbamate compound (1). When a divalent or tetravalent palladium catalyst is used as the palladium catalyst, it is preferable to use as a zerovalent palladium catalyst after reduction by an ordinary method.

A commercially available hydrogen gas can be typically used for the hydrogen.

The reduction reaction is carried out typically in the presence of a solvent.

Examples of the solvent include an aliphatic hydrocarbon solvent such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; an aromatic solvent such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; an ether solvent such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; an alcohol solvent such as methanol, ethanol, 1-propanol, 2-propanol, n-butyl alcohol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; an ester solvent such as ethyl acetate, propyl acetate, isopropyl acetate, butyl acetate, isobutyl acetate, t-butyl acetate, amyl acetate, and isoamyl acetate; and water.

These solvents may be used either alone or simultaneously as a combination of two or more kinds.

Preferred among these is an alcohol solvent or water; more preferable is at least one solvent selected from the group consisting of water, methanol, ethanol and 2-propanol; and water is still more preferable.

The amount of use of the solvent is typically 0.1 to 30 L, preferably 1 to 10 L, relative to 1 kg of the pyridin-3-ylcarbamate compound (3). Further, it is possible to use a carboxylic acid compound or phosphoric acid as a solvent as will be described later. In this case, the aforesaid solvent may be used or may not be used.

In order to improve the reaction speed or yield, the reduction reaction is preferably carried out in the presence of a carboxylic acid compound or phosphoric acid. Examples of the carboxylic acid compound include acetic acid, propionic acid, oxalic acid, malic acid, succinic acid, citric acid and lactic acid; and acetic acid is preferable. Enough is one time by mol of the use of the carboxylic acid compound or the phosphoric acid relative to the pyridin-3-ylcarbamate compound (3). Though an excessive amount can be used so as to serve also as a solvent, the amount is preferably 1.1 to 10 times by mol in consideration of economic property.

When water is used as a solvent, in order to improve the reaction speed or yield, the reaction is preferably carried out under a condition such that the pH value of the water layer within a range from 1 to 7, more preferably within a range from 2 to 6. It is preferable to adjust to such a pH range with use of the aforesaid carboxylic acid compound or phosphoric acid.

The reaction temperature is typically 20°C to 150°C, preferably 50°C to 120°C. The hydrogen pressure at the time of reaction is typically 0.1 to 5 MPa, preferably 0.2 to 1 MPa. The reaction time is typically 1 to 24 hours, though it depends on the reaction temperature, the amount of use of the reaction reagents, and the like. Progress of the reaction can be confirmed by ordinary means such as gas chromatography or high-speed liquid chromatography.

The order of mixing the reaction reagents is not particularly limited. Examples of the method include a method in which a mixture of a pyridin-3-ylcarbamate compound (3) and a solvent is added into a mixture of carboxylic acid compound or phosphoric acid, a palladium catalyst, and a solvent in a hydrogen atmosphere; and a method in which hydrogen is introduced to a mixture of carboxylic acid compound or phosphoric acid, a pyridin-3-ylcarbamate compound (3), a palladium catalyst, and a solvent. The method in which hydrogen is introduced to a mixture of carboxylic acid compound or phosphoric acid, a pyridin-3-ylcarbamate compound (3), a palladium catalyst, and a solvent is preferable.

The mixture after the reaction is finished contains a piperidin-3-ylcarbamate compound represented by the above formula (1) (hereafter abbreviated as piperidin-3-ylcarbamate compound (1)). The mixture may be subjected to an ordinary isolation process such as distillation or crystallization after being subjected to an ordinary post-treatment such as filtration, extraction, or water-washing, whereby the piperidin-3-ylcarbamate compound (1) can be isolated. Further, the obtained piperidin-3-ylcarbamate compound (1) may be purified by being subjected to an ordinary purification process such as recrystallization; extraction purification; distillation; adsorption process with activated carbon, silica, alumina, or the like; a chromatography method such as silica gel column chromatography; or the like. Piperidin-3-ylcarbamate compound (1) obtained in this manner are ethyl piperidin-3-ylcarbamate, and t-butyl piperidin-3-ylcarbamate.

The above piperidin-3-ylcarbamate compound (1) is typically a racemic form. The racemic form is typically an RS mixture of the piperidin-3-ylcarbamate compound (1), and is a mixture containing both of the R form and the S form among the mirror image isomers.

By performing optical resolution of the racemic form of the piperidin-3-ylcarbamate compound (1), it is also possible to obtain a diastereomer salt (which may hereafter be abbreviated as diastereomer salt) of an optically active piperidin-3-ylcarbamate compound represented by the formula (2) 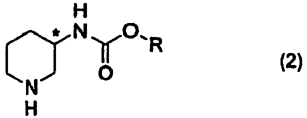 (in the formula, R has the same meaning as the above, and * represents that the carbon atom is an optically active center) (hereafter abbreviated as optically active piperidin-3-ylcarbamate compound (2)). Such optical resolution can be carried out typically by bringing the piperidin-3-ylcarbamate compound (1) and an optically active organic acid into contact in the presence of a solvent.

The optically active organic acid is an optically active mandelic acid. When R in the above formula (1) is an ethyl group or a t-butyl group, the optical resolution can be carried out efficiently by using an optically active mandelic acid.

The amount of use of the optically active organic acid is not particularly limited as long as the amount is 0.5 time by mol or more relative to the piperidin-3-ylcarbamate compound (1). In view of the yield and economic property, the range of 0.9 to 2 mol is preferable, and the range of 1.0 to 1.5 mol is more preferable.

Examples of the solvent used for optical resolution include an aliphatic hydrocarbon solvent such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and etroleum ether; an aromatic solvent such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; an ether solvent such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; an alcohol solvent such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; a nitrile solvent such as acetonitrile, propionitrile, or benzonitrile; an ester solvent such as methyl acetate, ethyl acetate, propyl acetate, isopropyl acetate, butyl acetate, isobutyl acetate, and t-butyl acetate; a ketone solvent such as acetone, methyl ethyl ketone, methyl isobutyl ketone, 2-pentanone, 3-pentanone, cyclopentanone, and cyclohexanone; a chlorinated aliphatic hydrocarbon solvent such as dichloromethane, chloroform, and 1,2-dichloroethane; a non-protonic polar solvent such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone, and 1,3-dimethyl-3,4,5,6-tetrahydro-2(1H)-pyridinone; and water.

These solvents may be used either alone or simultaneously as a combination of two or more kinds.

When optically active mandelic acid is used as the optically active organic acid, at least one solvent selected from the group consisting of a ketone solvent, an ester solvent, an alcohol solvent, and an ether solvent is preferable; a ketone solvent, an alcohol solvent, or an ether solvent is more preferable; methanol, ethanol, 2-propanol, 1-propanol, 1-butanol, methyl ethyl ketone, methyl isobutyl ketone, or tetrahydrofuran is still more preferable; and ethanol, 2-propanol, 1-butanol, or tetrahydrofuran is particularly preferable.

The amount of use of the solvent can be suitably selected in accordance with the solubility of the diastereomer salt. The amount is typically 1 to 50 L, preferably 3 to 30 L, relative to 1 kg of the piperidin-3-ylcarbamate compound (1).

The optical resolution is carried out typically by mixing the piperidin-3-ylcarbamate compound (1) with an optically active mandelic acid in the presence of a solvent, and the order of mixing is not particularly limited. When a crystal of the diastereomer salt is absent in the obtained mixture, the diastereomer salt may be crystallized by cooling the mixture as it is. When a crystal of the diastereomer salt is present in the obtained mixture, the mixture may be cooled as it is; however, it is preferable to crystallize the diastereomer salt by cooling after the crystal of the diastereomer salt is dissolved by heating the mixture, in view of the chemical purity or optical purity of the later-mentioned optically active piperidin-3-ylcarbamate compound (2), optically active 3-aminopiperidine, or salt thereof. In such crystallization of the diastereomer salt, a seed crystal of the diastereomer salt may be used.

The temperature for mixing the piperidin-3-ylcarbamate compound (1) with an optically active mandelic acid is not particularly limited, and is typically within a range higher than or equal to 0°C and lower than or equal to the boiling point of the solvent. When heating is carried out after mixing these, the heating is carried out typically up to a range higher than or equal to 30°C and lower than or equal to the boiling point of the solvent. The cooling temperature is typically within a range from 0 to 25°C, and it is preferable to cool gradually in view of the chemical purity and the optical purity of the obtained diastereomer salt.

The mixture obtained in this manner may be subjected, for example, to a solid-liquid separation process such as filtration or decantation, whereby the diastereomer salt can be taken out as a solid. Also, the liquid obtained by the above solid-liquid separation process typically contains a piperidin-3-ylcarbamate compound (2) that is rich in the mirror image isomer reverse to that constituting the diastereomer salt, so that an optically active piperidin-3-ylcarbamate compound (2) or a salt thereof can be taken out from the liquid by a conventional method.

The diastereomer salt that has been taken out may be subjected to a washing process or may be further subjected to a drying process. Typically for such a washing process, the same solvent as described above can be used. The condition for the drying process is typically within a range from 20°C to 80°C under an ordinary-pressure or reduced-pressure condition.

Typically, when acid or base is allowed to act on the obtained diastereomer salt at 0°C or higher and below 60°C, the optically active piperidin-3-ylcarbamate compound (2) or a salt thereof is preferentially obtained. When acid or base is allowed to act on the obtained diastereomer salt at 60°C or above and 150°C or below, the optically active 3-aminopiperidine or a salt thereof is preferentially obtained.

An RS mixture (racemate) of the piperidin-3-ylcarbamate compound (1) can be manufactured in accordance with an arbitrary known method. For example, it may be manufactured by performing nuclear reduction of ethyl pyridin-3-ylcarbamate or t-butyl pyridin-3-ylcarbamate obtained by ethyl-carbamation or t-butyl-carbamation of the amino group at 3-position of 3-aminopyridine, or by ethyl-carbamation or t-butyl-carbamation of the amino group at 3-position of the RS mixture of 3-aminopiperidine.

Hereafter, a method of performing optical resolution on the RS mixture of the piperidin-3-ylcarbamate compound (1) by using optically active mandelic acid will be described in more detail.

Typically, for the optically active mandelic acid, a commercially available one can be used. Not only the R-mandelic acid or the S-mandelic acid but also a mixture of these may be used as long as one of these is contained significantly in a greater amount. The optical purity thereof is preferably 90%ee or more, more preferably 95%ee or more, still more preferably 98%ee or more, and most preferably 100%ee. When an R body of the optically active piperidin-3-ylcarbamate compound represented by the above formula (2) (hereafter abbreviated as optically active piperidin-3-ylcarbamate compound (2)) is desired, it is preferable to use R-mandelic acid as the optically active mandelic acid. When an S form of the optically active piperidin-3-ylcarbamate compound (2) is desired, it is preferable to use S-mandelic acid as the optically active mandelic acid.

The amount of use of the optically active mandelic acid is not particularly limited as long as the amount is one mol multiple or more relative to the compound on the side with which it is desired to form a salt of optically active mandelic acid among the RS mixture of the piperidin-3-ylcarbamate compound (1) (R form in the case of using R-mandelic acid and S form in the case of using S-mandelic acid). The amount of use of the optically active mandelic acid in the case of using a racemic body as the RS mixture of the piperidin-3-ylcarbamate compound (1) may be typically 0.5 mol or more relative to one mol of the racemic form. The amount is preferably 0.9 to 2 mol, more preferably 1.0 to 1.5 mol, in view of the yield and economic property.

The contact of the RS mixture of the piperidin-3-ylcarbamate compound (1) with optically active mandelic acid is carried out typically in the presence of a solvent.

Examples of the solvent include an aliphatic hydrocarbon solvent such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; an aromatic solvent such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; an ether solvent such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, or diphenyl ether; an alcohol solvent such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; a nitrile solvent such as acetonitrile, propionitrile, and benzonitrile; a chlorinated aliphatic hydrocarbon solvent such as dichloromethane, chloroform, and 1,2-dichloroethane; an ester solvent such as methyl acetate, ethyl acetate, propyl acetate, isopropyl acetate, butyl acetate, isobutyl acetate, t-butyl acetate, amyl acetate, isoamyl acetate, hexyl acetate, methyl propionate, ethyl propionate, propyl propionate, and isopropyl propionate; a ketone solvent such as acetone, methyl ethyl ketone, methyl propyl ketone, methyl isopropyl ketone, methyl butyl ketone, methyl isobutyl ketone, diethyl ketone, cyclopentanone, and cyclohexanone; a non-protonic polar solvent such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone, 1,3-dimethyl-3,4,5,6-tetrahydro-2(1H)-pyridinone, and acetone; a carboxylic acid solvent such as formic acid, acetic acid, and propionic acid; and water.

These solvents may be used either alone or simultaneously as a combination of two or more kinds.

At least one solvent selected from the group consisting of a ketone solvent, an ester solvent, an alcohol solvent, and an ether solvent is preferable; a ketone solvent, an alcohol solvent, and an ether solvent is more preferable; methanol, ethanol, 2-propanol, 1-propanol, 1-butanol, methyl ethyl ketone, methyl isobutyl ketone, or tetrahydrofuran is still more preferable; and ethanol, 2-propanol, 1-butanol, or tetrahydrofuran is particularly preferable.

The amount of use of the solvent may be suitably selected in accordance with the solubility of the obtained salt made of optically active piperidine-3-ylcarbamate compound (2) and optically active mandelic acid (which may hereafter be abbreviated as a diastereomer salt). The amount is typically 1 to 50 L, preferably 3 to 30 L, relative to 1 kg of the RS mixture of the piperidin-3-ylcarbamate compound (1).

The contact of the RS mixture of the piperidin-3-ylcarbamate compound (1) with optically active mandelic acid is carried out by mixing these in the presence of a solvent, and the order of mixing is not particularly limited. When a crystal of the diastereomer salt is absent in the obtained mixture, the diastereomer salt may be crystallized by cooling the mixture as it is. When a crystal of the diastereomer salt is present in the mixture obtained by the above contact, the mixture may be cooled as it is; however, it is preferable to crystallize the diastereomer salt by cooling after the crystal of the diastereomer salt is dissolved by heating the mixture, in view of the chemical purity or optical purity of the finally obtained optically active piperidin-3-ylcarbamate compound (2) or a salt thereof. In such crystallization of the diastereomer salt, a seed crystal of the diastereomer salt may be used.

The temperature for mixing the RS mixture of the piperidin-3-ylcarbamate compound (1) with optically active mandelic acid is not particularly limited, and is typically within a range higher than or equal to 0°C and lower than or equal to the boiling point of the solvent. When heating is carried out after mixing these, the heating is carried out typically up to a range higher than or equal to 30°C and lower than or equal to the boiling point of the solvent. The cooling temperature is typically within a range from 0 to 25°C, and it is preferable to cool gradually in view of the chemical purity and the optical purity of the obtained diastereomer salt.

The mixture obtained in this manner may be subjected, for example, to a solid-liquid separation process such as filtration or decantation, whereby the diastereomer salt can be taken out as a solid. Such a diastereomer salt is a novel compound. Further, the liquid obtained by the above solid-liquid separation process typically contains a piperidin-3-ylcarbamate compound (2) that is rich in the mirror image isomer reverse to that constituting the diastereomer salt, so that an optically active piperidin-3-ylcarbamate compound (2) or a salt thereof can be taken out from the liquid by a conventional method.

An acid or base may be allowed to act on the diastereomer salt that has been taken out as it is; however, it is preferable to treat with an acid or base after the diastereomer salt is subjected to a washing process, in view of the chemical purity or optical purity of the finally obtained optically active piperidin-3-ylcarbamate compound (2). Typically for such a washing process, the same solvent as described above can be used. Preferably, a drying process is further carried out after the washing. The condition for the drying process is typically within a range from 20°C to 80°C under an ordinary-pressure or reduced-pressure condition.

Typically, the diastereomer salt obtained in this manner is a salt made of ethyl (R)-piperidin-3-ylcarbamate and R-mandelic acid or a salt made of t-butyl (R)-piperidin-3-ylcarbamate and R-mandelic acid in the case of using R-mandelic acid, and is a salt made of ethyl (S)-piperidin-3-ylcarbamate and S-mandelic acid or a salt made of t-butyl (S)-piperidin-3-ylcarbamate and S-mandelic acid in the case of using S-mandelic acid.

Typically, when an acid or base is allowed to act on the diastereomer salt, an optically active piperidin-3-ylcarbamate compound (2) or a salt thereof is obtained.

The acid that is allowed to act on the diastereomer salt may be one having a stronger acidity than mandelic acid, and may be, for example, a mineral acid such as hydrochloric acid, phosphoric acid, or sulfuric acid, an organic acid such as paratoluenesulfonic acid, benzenesulfonic acid, or camphorsulfonic acid, or the like. The acid is preferably hydrochloric acid. For such an acid, a commercially available one may be used as it is or as a solution of the later-mentioned solvent.

The amount of use of the acid is not particularly limited as long as the amount is one mol or more relative to one mol of the optically active piperidin-3-ylcarbamate compound (2) constituting the diastereomer salt.

Typically, the acid is allowed to act on the diastereomer salt in the presence of a solvent.

Examples of the solvent include an aliphatic hydrocarbon solvent such as pentane, hexane, isohexane, heptane, isoheptane, octane, isooctane, nonane, isononane, decane, isodecane, undecane, dodecane, cyclopentane, cyclohexane, methylcyclohexane, t-butylcyclohexane, and petroleum ether; an aromatic solvent such as benzene, toluene, ethylbenzene, isopropylbenzene, t-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene, and 1,2,4-trichlorobenzene; an ether solvent such as tetrahydrofuran, methyltetrahydrofuran, diethyl ether, dipropyl ether, diisopropyl ether, dibutyl ether, dipentyl ether, dihexyl ether, diheptyl ether, dioctyl ether, t-butyl methyl ether, cyclopentyl methyl ether, 1,2-dimethoxyethane, diethylene glycol dimethyl ether, anisole, and diphenyl ether; an alcohol solvent such as methanol, ethanol, 1-propanol, 2-propanol, 1-butanol, isobutyl alcohol, t-butyl alcohol, 1-pentanol, 2-pentanol, isopentyl alcohol, 1-hexanol, 2-hexanol, isohexyl alcohol, 1-heptanol, 2-heptanol, 3-heptanol, isoheptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-t-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether, and diethylene glycol mono-t-butyl ether; a nitrile solvent such as acetonitrile, propionitrile, and benzonitrile; an ester solvent such as ethyl acetate, propyl acetate, isopropyl acetate, butyl acetate, isobutyl acetate, t-butyl acetate, amyl acetate, and isoamyl acetate; a ketone solvent such as acetone, methyl ethyl ketone, methyl isopropyl ketone, methyl isobutyl ketone, cyclopentanone, and cyclohexanone; a chlorinated aliphatic hydrocarbon solvent such as dichloromethane, chloroform, and 1,2-dichloroethane; a carboxylic acid solvent such as formic acid, acetic acid, and propionic acid; and water.

These solvents may be used either alone or simultaneously as a combination of two or more kinds. Preferred among these is an aromatic solvent, an alcohol solvent or water. Toluene, xylene, methanol, ethanol, 2-propanol, 1-propanol, 1-butanol, or water is more preferable, and water is still more preferable.

The amount of use of the solvent is typically 1 to 50 L, preferably 3 to 30 L, relative to 1 kg of the diastereomer salt.

The diastereomer salt and the acid may be mixed typically at 0°C to 40°C, preferably at 0°C to 30°C, and the order of mixing these is not particularly limited.

The action time is not particularly limited, and is typically within a range from 1 minute to 24 hours.

When a salt of the optically active piperidin-3-ylcarbamate compound (2) and the acid put to use is deposited in the obtained mixture, the salt can be taken out by subjecting the mixture, for example, to a solid-liquid separation process such as filtration or decantation as it is. Also, when the deposition of the salt is insufficient or when the salt is not deposited, the salt may be crystallized, for example, by concentrating the mixture, mixing the mixture with a solvent that hardly dissolves the salt, heating the mixture, or cooling the mixture, and the salt may be taken out by subjecting the obtained mixture, for example, to a solid-liquid separation process such as filtration or decantation. The obtained salt may be further purified, for example, by ordinary means such as recrystallization, or may be made free in the same manner as in the later-mentioned case of allowing a base to act. Also, the filtrate obtained by the above-described solid-liquid separation process typically contains optically active mandelic acid, and the optically active mandelic acid can be collected from the filtrate by ordinary means so as to be recycled for use in the present invention.

Examples of the base, which is allowed to act on the diastereomer salt, include an alkali metal hydroxide such as potassium hydroxide and sodium hydroxide; an alkali metal carbonate such as sodium carbonate and potassium carbonate; and an alkali metal alcoholate such as sodium methylate, sodium ethylate, potassium methylate, and potassium ethylate. An alkali metal hydroxide is preferable, and sodium hydroxide is more preferable. For these bases, a commercially available one can be used, or these bases can be used as a solution of the later-mentioned solvent.

The amount of use of the base is not particularly limited as long as the amount is one mol or more relative to one mol of the optically active mandelic acid constituting the diastereomer salt.

Typically, a base is allowed to act on the diastereomer salt in the presence of a solvent. Examples of the solvent include an alcohol solvent such as methanol, ethanol, 2-propanol, 1-propanol, and 1-butanol; an ether solvent such as diethyl ether, t-butyl methyl ether, methyl isobutyl ether, diisopropyl ether, methyl cyclopentyl ether, and 1,2-dimethoxyethane; an aromatic solvent such as toluene, xylene, and chlorobenzene; an aliphatic hydrocarbon solvent such as hexane and cyclohexane; a ketone solvent such as methyl ethyl ketone and methyl isobutyl ketone; an ester solvent such as ethyl acetate and t-butyl acetate; a halogenated aliphatic hydrocarbon solvent such as dichloromethane; and water. These solvents may be used either alone or as a combination of two or more kinds. When an inorganic base such as an alkali metal hydroxide or an alkali metal carbonate is used as the base, it is preferable to use water alone or an organic solvent having a low compatibility with water (an ether solvent, an aromatic solvent, an aliphatic hydrocarbon solvent, a ketone solvent, an ester solvent, or a halogenated hydrocarbon solvent described above) and water simultaneously. An alcohol solvent, water, or a mixed solvent of these is preferable, and 1-butanol, water, or a mixed solvent of these is preferable.

The amount of use of the solvent is typically 1 to 50 L, preferably 3 to 30 L, relative to 1 kg of the diastereomer salt.

The diastereomer salt and the base may be mixed typically at 0°C to 60°C, preferably at 10°C to 30°C, and the order of mixing these is not particularly limited.

The action time is not particularly limited, and is typically within a range from 1 minute to 24 hours.

For example, an organic layer containing an optically active piperidin-3-ylcarbamate compound (2) can be obtained by adding a base to a mixture of water and a diastereomer salt, allowing the water layer of the mixture to become basic (typically to a pH value of 8.5 or higher), allowing the resultant to act at a predetermined temperature, thereafter adding an organic solvent having a low compatibility with water to the obtained mixture, and performing a liquid-separation process. When the organic layer is subjected to a concentration process after performing a water-washing process in accordance with the needs, the optically active piperidin-3-ylcarbamate compound (2) can be isolated. Also, when an alkali metal alcoholate is used as the base and an alcohol solvent is used as the solvent, an alkali metal salt of optically active mandelic acid is typically deposited. By separating this by filtration and performing a concentration process on the obtained solution, the optically active piperidin-3-ylcarbamate compound (2) can be isolated. The obtained optically active piperidin-3-ylcarbamate compound (2) can be further purified, for example, by ordinary means such as distillation, recrystallization, or column chromatography. The optically active piperidin-3-ylcarbamate compound (2) can be taken out as an acid-added salt. The water layer obtained by the above-described liquid-separation process contains optically active mandelic acid, and the optically active mandelic acid can be collected from the water layer by conventional means and can be recycled for use in the present invention. Further, the optically active mandelic acid can be collected from the alkali metal salt of the optically active mandelic acid separated by filtration in the above by conventional means and can be recycled for use in the present invention.

The optical purity of the optically active piperidin-3-ylcarbamate compound (2) obtained in this manner is typically 90%ee or higher though this depends on the optical purity of the optically active mandelic acid that has been put to use.

### EXAMPLES

### Reference Example 1: Manufacturing isopropyl pyridin-3-ylcarbamate

Into a solution obtained by dissolving 50.0 g (0.53 mol) of 3-aminopyridine and 8.94 g (0.11 mol) of sodium hydrogencarbonate in 150 mL of water, 75.7 g (0.61 mol) of isopropyl chlorocarbonate and 230 mL of a 15 wt% aqueous solution of potassium hydroxide were added dropwise in parallel over two hours. The inner temperature of the mixture during the dropwise addition was maintained to be 0 to 10°C, and the pH value was maintained to be 7 to 8. After the dropwise addition was finished, the obtained mixture was stirred at room temperature for one hour, and the deposited crystals were filtered and washed with 200 mL of water. By drying the obtained crystals, 89.3 g of isopropyl pyridin-3-ylcarbamate was obtained, with a yield of 93.3%.

### Reference Example 2: Manufacturing isopropyl piperidin-3-ylcarbamate

To a solution obtained by dissolving 89.3 g (0.50 mol) of isopropyl pyridin-3-ylcarbamate obtained in Reference Example 1 in 178.6 g (2. 97 mol) of acetic acid, 17. 9 g of palladium carbon (5%) was added, and the resultant was stirred for 14 hours at a hydrogen pressure of 0.5 MPa and at 70°C. After the reaction was finished, palladium carbon was separated by filtration to obtain a reaction solution; palladium carbon was washed with 225 mL of water to obtain a washing liquid; and the aforesaid reaction solution and the washing liquid were mixed. The obtained solution was dropwise added into a solution obtained in advance by dissolving 119 g (2.98 mol) of sodium hydroxide into 129 mL of water. The inner temperature of the mixture during the dropwise addition was set to be 0 to 10°C. The obtained mixture was subjected to an extraction process with 180 mL of t-butyl methyl ether, and the obtained organic layer was dried over magnesium sulfate. The magnesium sulfate was filtered off and the obtained solution was subjected to a concentration process to obtain 85.0 g of isopropyl piperidin-3-ylcarbamate as a yellow white crystal, with a yield of 92.1%.
¹H-NMR (DMSO-d₆, 400MHz) δ ppm: 6.85 (1H, d, J=7.8Hz), 4.79-4.68 (1H, m), 3.30-3.15 (1H, m), 2.85 (1H, d-like, J=11.7Hz), 2.69 (1H, d-like, J=12.2Hz), 2.30 (1H, t-like, J=10.2Hz), 2.19 (1H, t-like, J=11.2Hz), 2.10-1.95 (1H, m), 1.80-1.68 (1H, m), 1.58-1.49 (1H, m) 1.35-1.18 (2H, m), 1.14 (6H, d, J=6.3Hz) ¹³C-NMR (DMSO-d₆, 400MHz) δ ppm: 155.0, 66.2, 51.6, 47.8, 45.6, 31.0, 25.2, 22.1

### Reference Example 3: Manufacturing isopropyl piperidin-3-ylcarbamate

Into 90 mL of water, 60 g (0.33 mol) of isopropyl pyridin-3-ylcarbamate obtained in the same manner as in Reference Example 1 was suspended; 30 g (0.50 mol) of acetic acid was added to the resultant; 3.0 g of palladium carbon (5%) was added; and the obtained mixture was stirred for 23 hours at a hydrogen pressure of 0. 5 MPa and at 90°C. After the reaction was finished, palladium carbon was separated by filtration by adding 100 mL of 1-butanol to the reaction mixture, so as to obtain a reaction solution. Palladium carbon was washed with 20 mL of 1-butanol to obtain a washing liquid, and the aforesaid reaction solution and the washing liquid were mixed. Into the obtained solution, a solution obtained by dissolving 20.0 g (0.50 mol) of sodium hydroxide in 47 mL of water was added dropwise at 20 to 25°C and stirred, and an organic layer was obtained by a liquid-separation process. The water layer was extracted with 120 mL of 1-butanol, and the obtained organic layer and the organic layer obtained in advance were mixed. The obtained solution was washed with 90 mL of water, and further washed twice with 60 mL of water. By performing a concentration process on the obtained solution, 56.0 g of isopropyl piperidin-3-ylcarbamate was obtained as a yellow white crystal, with a yield of 90.4% (based on isopropyl pyridin-3-ylcarbamate).

### Reference Examples 4 to 8: Manufacturing isopropyl piperidin-3-ylcarbamate

Into 3 mL of water, 1.0 g (5.55 mmol) of isopropyl pyridin-3-ylcarbamate was suspended; the acid shown in Table 1 (the amount is shown as a mol multiple relative to isopropyl pyridin-3-ylcarbamate) was added to the resultant; 0.1 g of palladium carbon (10%) was further added; and the obtained mixture was allowed to react for 8 hours at a hydrogen pressure of 0.6 MPa and at 70 to 75°C. After the reaction was finished, the reaction mixture was neutralized with use of a saturated aqueous solution of sodium carbonate at 20 to 30°C; an organic layer obtained by performing extraction on the mixture with 4-methyl-2-pentanone was analyzed by gas chromatography, so as to determine an area percentage (described as GC SP in Table 1) of isopropyl pyridin-3-ylcarbamate (described as source material in Table 1) and isopropyl piperidin-3-ylcarbamate (described as object product in Table 1). The results are shown in Table 1 together with Reference Examples 2 and 3.

**Table 1**

| | Kind of acid | Amount of use of acid (times by mol) | pH | GC SP (%) | |
|---|---|---|---|---|---|
| | | | | Source material | Object product |
| Reference Example 2 | Acetic acid | 6 | 3.6 | 0.1 | 96.1 |
| Reference Example 3 | Acetic acid | 1.5 | 5.7 | 0.7 | 94.8 |
| Reference Example 4 | Acetic acid | 1.0 | 5.6 | 79.6 | 19.2 |
| Reference Example 5 | Phosphoric acid | 1.5 | 2.6 | 0.5 | 98.7 |
| Reference Example 6 | L-oxalic acid | 1.5 | 2.8 | 0.2 | 99.6 |
| Reference Example 7 | Citric acid monohydrate | 1.5 | 3.0 | 2.3 | 95.5 |
| Reference Example 8 | L-lactic acid | 1.5 | 4.6 | 1.3 | 98.2 |

### <gas chromatography analysis condition>

| | |
|---|---|
| column : | DB-5 manufactured by J&W Co., Ltd., 0.53mm X 30 m, 1 µm |
| gasifying chamber : | 250°C |
| detector : | 280°C (FID) |
| carrier gas : | helium, 80 cm/second |
| temperature : | 100°C (5 minutes) - 12°C/minute - 280°C (10 minutes) |
| split ratio : | 3.0 |

### <holding time>

isopropyl pyridin-3-ylcarbamate (source material) : 12.3 minutes

isopropyl piperidin-3-ylcarbamate (object product) : 11.3 minutes

### Reference Example 9: Manufacturing ethyl pyridin-3-ylcarbamate

Into a solution obtained by dissolving 100 g (1.06 mol) of 3-aminopyridine into 650 ml of water, 121.1 g (1.12 mol) of ethyl chlorocarbonate and 240 g of a 20 wt% aqueous solution of sodium hydroxide were dropwise added in parallel in two hours. The inner temperature of the mixture during the dropwise addition was maintained to be 0 to 15°C, and the pH value was maintained to be 7 to 8.5. After the dropwise addition was finished, the obtained mixture was stirred at 10°C for two hours, and the deposited crystals were filtered and washed with 500 ml of water. By drying the obtained crystals, 148.5 g of ethyl pyridin-3-ylcarbamate was obtained, with a yield of 84.1%. Reference Example 10: Manufacturing ethyl piperidin-3-ylcarbamate

Into a solution obtained by dissolving 145 g of ethyl pyridin-3-ylcarbamate obtained in Reference Example 9 in 157 g (2.61 mol) of acetic acid and 145 ml of water, 14.5 g of palladium carbon (10%) was added, and the resultant was stirred for 6 hours at a hydrogen pressure of 0.5 MPa and at 70 to 90°C. After the reaction was finished, palladium carbon was separated by filtration to obtain a reaction solution; palladium carbon was washed with 300 ml of 1-butanol to obtain a washing liquid; and the aforesaid reaction solution and the washing liquid were mixed. Into the obtained solution, a solution obtained in advance by dissolving 105 g (2.63 mol) of sodium hydroxide in 244 ml of water was added dropwise. The inner temperature of the mixture during the dropwise addition was set to be 0 to 30°C. The obtained mixture was subjected to a liquid-separation process, and the organic layer was washed with 100 g of a 20 wt% aqueous solution of sodium chloride. Thereafter, the organic layer was concentrated and dissolved into 500 ml of 2-propanol. By performing a concentration process on the solution obtained by filtering insoluble substances, 141.9 g of ethyl piperidin-3-ylcarbamate was obtained as a faint yellow white crystal, with a yield of 94.4%.

### Reference Example 11: Manufacturing propyl pyridin-3-ylcarbamate

Into a solution obtained by dissolving 42.2 g (0.45 mol) of 3-aminopyridine into 148 ml of water, 54.9 g (0.45 mol) of propyl chlorocarbonate and 100 g of a 20 wt% aqueous solution of sodium hydroxide were added dropwise in parallel in two hours. The inner temperature of the mixture during the dropwise addition was maintained to be 0 to 15°C, and the pH value was maintained to be 7 to 8.5. After the dropwise addition was finished, the obtained mixture was stirred at room temperature for one hour, and the deposited crystals were filtered and washed with 300 ml of water. By drying the obtained crystals, 64.8 g of propyl pyridin-3-ylcarbamate was obtained, with a yield of 80.3%.

### Reference Example 12: Manufacturing propyl piperidin-3-ylcarbamate

Into a solution obtained by dissolving 60.0 g of propyl pyridin-3-ylcarbamate obtained in Reference Example 11 in 100 g (1. 67 mol) of acetic acid and 100 ml of water, 6. 0 g of palladium carbon (5%) was added, and the resultant was stirred for 8 hours at a hydrogen pressure of 0.5 MPa and at 70 to 85°C. After the reaction was finished, palladium carbon was separated by filtration to obtain a reaction solution; palladium carbon was washed with 180 ml of 1-butanol to obtain a washing liquid; and the aforesaid reaction solution and the washing liquid were mixed. Into the obtained solution, a solution obtained in advance by dissolving 66.6 g (1.67 mol) of sodium hydroxide in 200 ml of water was added dropwise. The inner temperature of the mixture during the dropwise addition was set to be 0 to 30°C. The obtained mixture was subjected to a liquid-separation process, and the organic layer was washed with 100 g of a 20 wt% aqueous solution of sodium chloride. Thereafter, the organic layer was concentrated and dissolved into 300 ml of 2-propanol. By performing a concentration process on the solution obtained by filtering insoluble substances, 59.1 g of propyl piperidin-3-ylcarbamate was obtained as a faint yellow crystal, with a yield of 95.3%.
¹H-NMR (DMSO-d₆, 400MHz)δ ppm: 6.95 (1H, d, J=8Hz), 3.87 (2H, t, J=7Hz), 3.28-3.26 (1H, m), 2.88 (1H, d-like), 2.72 (1H, d-like), 2.33 (1H, t-like), 2.23 (1H, t-like), 1.76-1.74 (1H, m), 1.59-1.50 (3H, m), 1.34-1.24 (2H, m), 0.88 (3H, t, J=7Hz) ¹³C-NMR (DMSO-d₆, 400MHz) δ ppm: 155.5, 65.0, 51.5, 47.8, 45.6, 31.0, 25.2, 22.1, 10.3

### Reference Example 13: Manufacturing isobutyl pyridin-3-ylcarbamate

Into a solution obtained by dissolving 16.4 g (0.17 mol) of 3-aminopyridine in 100 ml of water, 25.0 g (0.18 mol) of isobutyl chlorocarbonate and 100 g of a 15 wt% aqueous solution of sodium hydroxide were added dropwise in parallel over two hours. The inner temperature of the mixture during the dropwise addition was maintained to be 0 to 17°C, and the pH value was maintained to be 7 to 8.5. After the dropwise addition was finished, the obtained mixture was stirred overnight at room temperature, and the deposited crystals were filtered and washed with 300 ml of water. By drying the obtained crystals, 31.2 g of isobutyl pyridin-3-ylcarbamate was obtained, with a yield of 92.2%.

### Reference Example 14: Manufacturing isobutyl piperidin-3-ylcarbamate

Into a solution obtained by dissolving 30.4 g of the obtained isobutyl pyridin-3-ylcarbamate obtained in Reference Example 13 in 18.9 g (0.31 mol) of acetic acid, 45ml of water, and 90 ml of 1-butanol, 1. 5 g of palladium carbon (5%) was added, and the resultant was stirred for 5 hours at a hydrogen pressure of 0.5 MPa and at 70 to 85°C. After the reaction was finished, palladium carbon was separated by filtration to obtain a reaction solution; palladium carbon was washed with 30 ml of 1-butanol to obtain a washing liquid; and the aforesaid reaction solution and the washing liquid were mixed. Into the obtained solution, a solution obtained in advance by dissolving 12.6 g (0.31 mol) of sodium hydroxide in 29 ml of water was added dropwise. The inner temperature of the mixture during the dropwise addition was maintained to be 0 to 5°C. The obtained mixture was subjected to a liquid-separation process, and the organic layer was washed with 50 g of water for four times.

Thereafter, the organic layer was subjected to a concentration process to obtain 30.2 g of isobutyl piperidin-3-ylcarbamate as a yellow white crystal, with a yield of 95.9%. Reference Example 15: optical resolution of isopropyl piperidin-3-ylcarbamate

Into a solution obtained by dissolving 10 g (53.7 mmol) of isopropyl piperidin-3-ylcarbamate obtained in the same manner as in Reference Example 2 in 50 ml of ethanol, 8.46 g (56. 4 mmol) of L-oxalic acid was added, and the obtained solution was stirred at 40°C. When a seed crystal of a diastereomer salt of isopropyl (R)-piperidin-3-ylcarbamate and L-oxalic acid was added thereto, crystals were deposited. The temperature of the obtained mixture was raised to 65°C, and the mixture was stirred at the same temperature for one hour, whereby most part of the deposited crystals were dissolved, and a part thereof remained undissolved. The obtained mixture was cooled to room temperature by being left to stand while being stirred, and subsequently cooled to 0 to 5°C and stirred at the same temperature for 5 hours. Crystals were collected by filtration from the obtained mixture, and the crystals were washed with 20 ml of cool ethanol. By drying the obtained crystals, 6.95 g of a diastereomer salt of isopropyl (R)-piperidin-3-ylcarbamate and L-oxalic acid was obtained as a white crystal, with a yield of 38.5%. ¹H-NMR (DMSO-d₆, 400MHz)δ ppm: 7.24 (1H, d, J=8Hz), 4.80-4.72 (1H, m), 3.91 (2H, s), 3.60 (1H, br), 3.18 (1H, dd, J=4.12Hz), 3.09 (1H, d, J=13Hz), 2.72 (1H, t, J=11Hz), 2.59 (1H, t, J=12Hz), 1.82-1.77 (2H, m), 1.63-1.54 (1H, m), 1.43-1.31(1H, m), 1.17 (6H, d J=6Hz) ¹³C-NMR (DMSO-d₆, 400MHz) δ ppm: 174.5, 155.0, 71.8, 66.9, 46.6, 44.6, 42.7, 28.4, 22.0, 20.7

With use of triethylamine, isopropyl piperidin-3-ylcarbamate was taken out from the diastereomer salt, and this was derivatized with use of 3,5-dinitrobenzoyl chloride and analyzed by high-speed liquid chromatography, whereby the optical purity of the isopropyl piperidin-3-ylcarbamate in the diastereomer salt was 93.5%ee (R form).

### <optical purity analysis condition>

column: CHIRALCEL AS-RH (4.6 * 150 mm, 5 µm)
mobile phase: A = water, B = acetonitrile, A/B = 70/30
flow rate: 1.0 ml/minute
detector: UV 254 nm
holding time: S form = 19.1 minutes, R form = 31.5 minutes Reference Example 16: optical resolution of isopropyl piperidin-3-ylcarbamate

Into a solution obtained by dissolving 3.0 g (16.1 mmol) of isopropyl piperidin-3-ylcarbamate obtained in the same manner as in Reference Example 2 in 15 ml of a mixed solvent of methanol/1-butanol (1/1 (volume/volume)), 2.78 g (18.5 mmol) of L-oxalic acid was added, and the obtained solution was stirred at 40°C. When a seed crystal of a diastereomer salt of isopropyl (R) -piperidin-3-ylcarbamate and L-oxalic acid was added thereto, crystals were deposited. After the mixture was stirred at the same temperature for one hour, the resultant was cooled to room temperature by being left to stand while being stirred, and then stirred at the same temperature for 3 hours. Subsequently, the resultant was cooled to 10°C and stirred at the same temperature for 22 hours. Crystals were collected by filtration from the obtained mixture, and the crystals were washed with 5 ml of ethanol. By drying the obtained crystals, 1.96 g of a diastereomer salt of isopropyl (R)-piperidin-3-ylcarbamate and L-oxalic acid was obtained as a white crystal, with a yield of 36.2%.

This was analyzed by high-speed liquid chromatography in the same manner as in Example 9, whereby the optical purity of the isopropyl piperidin-3-ylcarbamate in the diastereomer salt was 96.8%ee (R form).

### Reference Examples 17 to 19: optical resolution of isopropyl piperidin-3-ylcarbamate

In Reference Examples 17 to 19, operation was carried out in the same manner as in Reference Example 16 except that the solvents described in Table 2 were used in place of methanol/1-butanol mixed solvent (1/1 (volume/volume)). The results are shown in Table 2.

### Reference Example 20: optical resolution of ethyl piperidin-3-ylcarbamate

In Reference Example 20, operation was carried out in the same manner as in Reference Example 16 except that 2.0 g (11.6 mmol) of ethyl piperidin-3-ylcarbamate obtained in the same manner as in Reference Example 10 was used in place of 3.0 g (16.1 mmol) of isopropyl piperidin-3-ylcarbamate obtained in the same manner as in Reference Example 2 and that methanol was used in place of methanol/1-butanol mixed solvent (1/1 (volume/volume)), with a result that 1.15 g of a diastereomer salt of ethyl (R)-piperidin-3-ylcarbamate and L-oxalic acid was obtained as a white crystal, with a yield of 30.7%. ¹H-NMR (DMSO-d₆, 400MHz) δ ppm: 7.35 (1H, d, J=7Hz), 4.01-3.97 (4H, m), 3.65 (1H, br), 3.19 (1H, d-like), 3.10 (1H, d-like), 2.74 (1H, t - like), 2.63 (1H, t-like), 1.90-1.70 (2H, m), 1.69-1.61 (1H, m), 1.94-1.39 (1H, m), 1.16 (3H, d, J=7Hz) ¹³C-NMR (DMSO-d6, 400MHz) δ ppm: 174.6, 155.4, 71.9, 59.8, 46.6, 44.7, 42.6, 28.5, 20.7, 14.6

With use of triethylamine, ethyl piperidin-3-ylcarbamate was taken out from the diastereomer salt, and this was derivatized with use of 3,5-dinitrobenzoyl chloride and analyzed by high-speed liquid chromatography, whereby the optical purity of the ethyl piperidin-3-ylcarbamate in the diastereomer salt was 94.3%ee (R form).

### <optical purity analysis condition>

column: CHIRALCEL AS-RH (4.6 * 150 mm, 5 µm)
mobile phase: A = water, B = acetonitrile, A/B = 65/35
flow rate: 1.0 ml/minute
detector: UV 254 nm
holding time: S form = 8.3 minutes, R form = 18.2 minutes Reference Example 20-2: Manufacturing ethyl (R)-piperidin-3-ylcarbamate

With 10 ml of water and 20 ml of ethyl acetate, 3.0 g (9.31 mmol, with an optical purity of 93.4%ee (R form)) of a diastereomer salt obtained by the same method as in Reference Example 20 was mixed. While maintaining the obtained mixture at 20 to 25°C, 2.02 g (19.09 mmol) of sodium carbonate was added, and the resultant was stirred. An organic layer was obtained by a liquid-separation process. The water layer was extracted with 20 ml of ethyl acetate, and the obtained organic layer and the organic layer obtained previously were mixed. The obtained organic layer was dried with use of anhydrous sodium sulfate. The sodium sulfate was separated by filtration, and the obtained solution was subjected to a concentration process, with a result that 1.46 g of ethyl (R)-piperidin-3-ylcarbamate was obtained as a white crystal, with a yield of 91%.
¹H-NMR (DMSO-d₆, 400MHz)δ ppm: 6.93 (1H, d, J=8Hz), 3.97-3.92 (2H, m), 3.33-3.18 (1H, m), 2.86 (1H, d-like), 2.70 (1H, d-like), 2.31 (1H, t-like), 2.20 (1H, t-like), 1.78-1.68(1H, m), 1.58-1.48 (1H, m), 1.38-1.20 (2H, m), 1.13 (3H, t, J=7Hz) ¹³C-NMR (DMSO-d₆, 400MHz) δ ppm: 155.4, 59.4, 51.3, 47.7, 45.5, 30.9, 25.0, 14.7

### Reference Example 21: optical resolution of ethyl piperidin-3-ylcarbamate

In Reference Example 21, operation was carried out in the same manner as in Reference Example 20 except that methanol/1-butanol mixed solvent (1/1 (volume/volume)) was used in place of methanol. The results are shown in Table 2. Reference Example 22: optical resolution of propyl piperidin-3-ylcarbamate

In Reference Example 22, operation was carried out in the same manner as in Reference Example 16 except that 1.0 g (5.4 mmol) of propyl piperidin-3-ylcarbamate obtained in the same manner as in Reference Example 12 was used in place of 3.0 g (16.1 mmol) of isopropyl piperidin-3-ylcarbamate obtained in the same manner as in Reference Example 2 and that ethanol was used in place of methanol/1-butanol mixed solvent (1/1 (volume/volume)), with a result that 0.46 g of a diastereomer salt of propyl (R)-piperidin-3-ylcarbamate and L-oxalic acid was obtained as a white crystal, with a yield of 25.5%. ¹H-NMR (DMSO-d₆, 400MHz) δ ppm: 7.34 (1H, d, J=7Hz), 3.97 (2H, s), 3.90 (2H, t, J=7Hz), 3.70-3.60 (1H, br), 3.19 (1H, d-like), 3.10 (1H, d-like), 2.73 (1H, t-like), 2.62(1H, t-like), 1.85-1.70 (2H, m), 1.65-1.50 (3H, m), 1.48-1.31 (1H, m), 0.88 (3H, t, J=7Hz)
¹³C-NMR (DMSO-d6, 400MHz) δ ppm: 174.4, 155.5, 71.8, 65.4, 46.7, 44.8, 42.7, 28.4, 22.0, 20.8, 10.3

With use of triethylamine, propyl piperidin-3-ylcarbamate was taken out from the diastereomer salt, and this was derivatized with use of 3,5-dinitrobenzoyl chloride and analyzed by high-speed liquid chromatography, whereby the optical purity of the propyl piperidin-3-ylcarbamate in the diastereomer salt was 90.0%ee (R form).

### <optical purity analysis condition>

column: CHIRALCEL AS-RH (4.6 * 150 mm, 5 µm)
mobile phase: A = water, B = acetonitrile, A/B = 65/35
flow rate: 1.0 ml/minute
detector: UV 254 nm
holding time: S form = 12.5 minutes, R form = 23.8 minutes Reference Example 23: optical resolution of isobutyl piperidin-3-ylcarbamate

In Reference Example 23, operation was carried out in the same manner as in Reference Example 16 except that 2.0 g (10.0 mmol) of isobutyl piperidin-3-ylcarbamate obtained in the same manner as in Reference Example 14 was used in place of 3.0 g (16.1 mmol) of isopropyl piperidin-3-ylcarbamate obtained in the same manner as in Reference Example 2 and that ethanol was used in place of methanol/1-butanol mixed solvent (1/1 (volume/volume)), with a result that 0.91 g of a diastereomer salt of isobutyl (R)-piperidin-3-ylcarbamate and L-oxalic acid was obtained as a white crystal, with an yield of 26.0%. ¹H-NMR (DMSO-d₆, 400MHz) δ ppm: 7.34 (1H, d, J=7Hz), 3.95 (2H, s), 3.74 (2H, d, J=6Hz), 3.67-3.60 (1H, br), 3.19 (1H, d-like), 3.10 (1H, d-like), 2.73 (1H, t-like), 2.62(1H, t-like), 1.85-1.70 (3H, m), 1.65-1.55 (1H, m), 1.48-1.35 (1H, m), 0.89 (6H, d, J=6Hz)
¹³C₋NMR (DMSO-d6, 400MHz) δ ppm:: 174.3, 155.5, 71.6, 69.8, 46.7, 44.8, 42.8, 28.4, 27.6, 20.9, 18.9

With use of triethylamine, isobutyl piperidin-3-ylcarbamate was taken out from the diastereomer salt, and this was derivatized with use of 3,5-dinitrobenzoyl chloride and analyzed by high-speed liquid chromatography, whereby the optical purity of the isobutyl piperidin-3-ylcarbamate in the diastereomer salt was 86.4%ee (R form).

### <optical purity analysis condition>

column: CHIRALCEL AS-RH (4.6 * 150 mm, 5 µm)
mobile phase: A = water, B = acetonitrile, A/B = 65/35
flow rate: 1.0 ml/minute
detector: UV 254 nm
holding time: S form = 19.8 minutes, R form = 37.7 minutes Reference Examples 24, 25: optical resolution of isobutyl piperidin-3-ylcarbamate

In Reference Examples 24, 25 operation was carried out in the same manner as in Reference Example 23 except that the solvents described in Table 2 were used in place of ethanol. The results are shown in Table 2.

The above results are shown in Table 2.

**TABLE 2**

| | R | Solvent | Yield | Optical purity of crystal |
|---|---|---|---|---|
| Reference Example 15 | Isopropyl group | Ethanol | 38.5% | 93.5%ee |
| Reference Example 16 | | Mixed solvent *1 | 36.2% | 96.8%ee |
| Reference Example 17 | | Mixed solvent *2 | 22.3% | 98.2%ee |
| Reference Example 18 | | 2-propanol | 46.5% | 84.1%ee |
| Reference Example 19 | | 1-butanol | 42.6% | 94.8%ee |
| Reference Example 20 | Ethyl group | Methanol | 30.7% | 94.3%ee |
| Reference Example 21 | | Mixed solvent *1 | 37.4% | 88.5%ee |
| Reference Example 22 | Propyl group | Ethanol | 25.5% | 90.0%ee |
| Reference Example 23 | Isobutyl group | Ethanol | 26.0% | 86.4%ee |
| Reference Example 24 | | Mixed solvent *3 | 28.6% | 83.3%ee |
| Reference Example 25 | | Mixed solvent *4 | 21.7% | 94.5%ee |

| | | | | |
|---|---|---|---|---|
| * mixed solvent 1: methanol / 1-butanol = 1/1 (volume/volume) * mixed solvent 2: methanol / 1-butanol = 2/1 (volume/volume) * mixed solvent 3: methanol / 1-butanol = 1/4 (volume/volume) * mixed solvent 4: methanol / 1-butanol = 1/2 (volume/volume) | | | | |

### Reference Example 26: Manufacturing t-butyl pyridin-3-ylcarbamate

Into a solution obtained by dissolving 80.0 g (0.85 mol) of 3-aminopyridine and 100 mL of an aqueous solution of 5wt% sodium hydrogencarbonate in 100 ml of methanol, a mixed solution of 213 g (0.98 mol) of di-t-butyl dicarbonate and 80 mL of methanol and 230 ml of a 20 wt% aqueous solution of sodium carbonate were added dropwise in parallel over two hours. The inner temperature of the mixture during the dropwise addition was maintained to be 0 to 10°C, and the pH value was maintained to be 7 to 8. After the dropwise addition was finished, the obtained mixture was stirred at room temperature for 12 hours, and the resultant was subjected to a concentration process under reduced pressure. To the concentration residue, 300 mL of water was added, and the deposited crystals were filtered and washed with 200 ml of water. By drying the obtained crystals, 146 g of t-butyl pyridin-3-ylcarbamate was obtained, with a yield of 88.4%.

### Reference Example 27: Manufacturing t-butyl piperidin-3-ylcarbamate

Into a solution obtained by dissolving 100 g (0.52 mol) of t-butyl pyridin-3-ylcarbamate obtained in Example 26 in 400 g (6.66 mol) of acetic acid, 30 g of palladium carbon (5%) was added, and the resultant was stirred for 12 hours at a hydrogen pressure of 0. 6 MPa and at 65°C. After the reaction was finished, palladium carbon was separated by filtration to obtain a reaction solution; palladium carbon was washed with 250 ml of water to obtain a washing liquid; and the aforesaid reaction solution and the washing liquid were mixed. The obtained solution was added dropwise into a solution obtained in advance by dissolving 266 g (6.65 mol) of sodium hydroxide in 500 ml of water. The inner temperature of the mixture during the dropwise addition was set to be 10 to 20°C. After 200 mL of water was further added thereto, the deposited crystals were filtered and washed with 400 ml of water. The obtained crystals were dried to obtain 76.2 g of t-butyl piperidin-3-ylcarbamate as a white crystal, with a yield of 73.8%.

### Reference Example 28: Manufacturing t-butyl pyridin-3-ylcarbamate

Into a solution obtained by dissolving 100.0 g (1.06 mol) of 3-aminopyridine in a mixed solvent of 300 ml of 2-propanol and 100 mL of water, a mixed solution of 266.7 g (1.22 mol) of di-t-butyl dicarbonate and 100 mL of 2-propanol was added dropwise over three hours. The inner temperature of the mixture during the dropwise addition was maintained to be 5 to 20°C. After the dropwise addition was finished, the obtained mixture was stirred at room temperature for 3 hours, and the resultant was subjected to a concentration process under reduced pressure. To the concentration residue, 200 mL of water was added. After the resultant was further subjected to a concentration process, 200 mL of water was added to the concentration residue, and the deposited crystals were filtered. The obtained crystals were washed with 200 ml of water to obtain 234.2 g of hydrous crystals of t-butyl pyridin-3-ylcarbamate. The hydrous crystals were analyzed by gas chromatography with a result that the content of t-butyl pyridin-3-ylcarbamate was 79.5 wt%, with a yield of 90.2%.

### Reference Example 29: Manufacturing t-butyl piperidin-3-ylcarbamate

Into 450 mL of 1-butanol, 195.2 g (pure moiety 155.2 g, 0.799 mol) of the hydrous crystals of t-butyl pyridin-3-ylcarbamate obtained in Reference Example 28 was dissolved, and the obtained solution was subjected to a concentration process under reduced pressure to remove 160 g of the solvent by distillation. To the obtained concentration residue, 310 mL of acetic acid and 17.4 g of palladium carbon (10%) were added, and the obtained mixture was stirred for 7 hours with a hydrogen pressure of 0.5 MPa and at 70°C. After the reaction was finished, palladium carbon was separated by filtration, so as to obtain a reaction solution. Palladium carbon was washed with 93 ml of 1-butanol to obtain a washing liquid, and the aforesaid reaction solution and the washing liquid were mixed. Into the obtained solution, 723 g (5.43 mol) of an aqueous solution of 30 wt% sodium hydroxide was added dropwise. The inner temperature of the mixture during the dropwise addition was maintained to be 10 to 30°C. An organic layer was obtained by a liquid-separation process. The water layer was extracted with 310 ml of 1-butanol; the obtained organic layer and the organic layer obtained previously were mixed; and then the resultant was twice washed with 155 mL of water. The obtained organic layer was concentrated under reduced pressure to obtain 489 g of a 1-butanol solution of t-butyl piperidin-3-ylcarbamate. The solution was analyzed by gas chromatography with a result that the content of t-butyl piperidin-3-ylcarbamate was 32.7%, with a yield of 99.7%. Example 30: optical resolution of t-butyl piperidin-3-ylcarbamate

A mixture was obtained by mixing 2.00 g (10.0 mmol) of t-butyl piperidin-3-ylcarbamate obtained in Reference Example 27, 1.55 g (10.2 mmol) of R-mandelic acid, and 10 ml of ethanol. When the obtained mixture was stirred at 70°C, a homogeneous solution was obtained. When the solution was cooled to room temperature, crystals were deposited. The crystals were collected by filtration, and the obtained crystals were washed with 5 ml of cool ethanol. By drying the obtained crystals, 1.28 g of a diastereomer salt of t-butyl (R)-piperidin-3-ylcarbamate and R-mandelic acid was obtained as a white crystal, with a yield of 36.6%.
¹H-NMR (DMSO-d₆, 400MHz) δ ppm: 7.36 (2H, d, J=7.2Hz), 7.25-7.13 (4H, m), 7.00 (1H, br), 4.59 (1H, s), 3.53 (1H, br), 3.09-2.97 (2H, m)), 2.64-2.49 (2H, m), 1.78-1.69 (2H, m), 1.53-1.21 (11H, m)
¹³C-NMR (DMSO-d₆, 400MHz) δ ppm: 175.1, 154.6, 143.1, 127.3, 126.2, 126.1, 78.0, 73.3, 47.0, 44.6, 42.8, 28.7, 28.2, 21.1

With use of triethylamine, t-butyl piperidin-3-ylcarbamate was taken out from the diastereomer salt, and this was derivatized with use of 3,5-dinitrobenzoyl chloride and analyzed by high-speed liquid chromatography, whereby the optical purity of the t-butyl piperidin-3-ylcarbamate in the diastereomer salt was 91.9%ee (R form).

### <optical purity analysis condition>

column: CHIRALCEL AS-RH (4.6 * 150 mm, 5 µm)
mobile phase: A = water, B = acetonitrile, A/B = 60/40
flow rate: 1.0 ml/minute
detector: UV 254 nm
holding time: S form = 10.4 minutes, R form = 15.6 minutes
Example 31: Manufacturing a diastereomer salt (optical resolution)

After 80 mL of 1-butanol and 160 mL of ethyl acetate were added to 489 g (pure moiety 159.7 g, 0.797 mol) of a 1-butanol solution of t-butyl piperidin-3-ylcarbamate obtained in Reference Example 29, 123.7 g (0.813 mmol) of R-mandelic acid was added. When the temperature was raised up to 62°C, a homogeneous solution was obtained. When the solution was cooled to 50°C, deposition of crystals was seen. After the mixture was stirred at 50°C for 3 hours, the resultant was cooled to 10°C and stirred for 5 hours at the same temperature. The deposited crystals were collected by filtration, and the crystals were washed with 160 ml of ethyl acetate. By drying the obtained crystals, 109. 0 g of a diastereomer salt of t-butyl (R)-piperidin-3-ylcarbamate and R-mandelic acid was obtained as a white crystal, with a yield of 38.8%.

Analysis was carried out with high-speed liquid chromatography by the same method as in Example 30, whereby the optical purity of t-butyl piperidin-3-ylcarbamate in the diastereomer salt was 88.5%ee (R form).

### Example 31-2: Purification of a diastereomer salt

To a mixed solvent of 160 mL of 1-butanol and 160 mL of ethyl acetate, 107 g (0.304 mol, optical purity 88.5%ee (R form)) of the diastereomer salt of t-butyl (R)-piperidin-3-ylcarbamate and R-mandelic acid obtained in Example 31 was added, and the obtained mixture was stirred at 75°C for 3 hours to obtain a homogeneous solution. When the solution was cooled down to 10°C and stirred at that temperature for 3 hours, crystals were deposited. The crystals were collected by filtration, and the crystals were washed with 107 ml of ethyl acetate. By drying the obtained crystals, 98.5 g of a diastereomer salt of t-butyl (R)-piperidin-3-ylcarbamate and R-mandelic acid was obtained as a white crystal, with a yield of 92.1%.

Analysis was carried out by the same method as in Example 30, whereby the optical purity of t-butyl piperidin-3-ylcarbamate in the diastereomer salt was 97.4%ee (R form).

### Example 31-3: Manufacturing t-butyl (R)-piperidin-3-ylcarbamate

A mixture was obtained by mixing 96.5 g (0.274 mol, optical purity 97.4%ee (R form)) of the diastereomer salt of t-butyl (R)-piperidin-3-ylcarbamate and R-mandelic acid obtained in Example 31-2 with 12.5 g of sodium chloride, 97 ml of water, and 193 ml of 1-butanol. While maintaining the obtained mixture at 10 to 30°C, 115 g (0.287 mol) of an aqueous solution of 10 wt% sodium hydroxide was added thereto, and the resultant was stirred. An organic layer was obtained by a liquid-separation process. The water layer was extracted with 97 ml of 1-butanol, and the obtained organic layer and the organic layer previously obtained were mixed. The obtained organic layer was washed twice with 97 mL of water, and the obtained organic layer was concentrated under reduced pressure. To the concentration residue, 297 mL of 4-methyl-2-pentanone was added, and the obtained mixture was partially concentrated, whereby crystals were deposited. The crystals were collected by filtration and washed with 107 ml of ethyl acetate. By drying the obtained crystals, 43.7 g of t-butyl (R)-piperidin-3-ylcarbamate was obtained as a white crystal, with a yield of 79.7%.

The obtained t-butyl piperidin-3-ylcarbamate was derivatized with use of 3,5-dinitrobenzoyl chloride and analyzed by high-speed liquid chromatography, whereby the optical purity of the t-butyl piperidin-3-ylcarbamate was 99.8%ee (R form).

### <optical purity analysis condition>

column: CHIRALCEL AS-RH (4.6 * 150 mm, 5 µm)
mobile phase: A = water, B = acetonitrile, A/B = 70/30
flow rate: 1.0 ml/minute
detector: UV 254 nm
holding time: S form = 10.4 minutes, R form = 15.6 minutes
Reference Example 32: Manufacturing ethyl pyridin-3-ylcarbamate

A mixture was obtained by mixing 30.0 g (0.32 mol) of 3-aminopyridine, 48.5 g (0.35 mol) of potassium carbonate, and 100 ml of acetone. While cooling the obtained suspension with ice, 36.3 g (0.34 mol) of ethyl chlorocarbonate was added dropwise thereto over 2 hours. After the dropwise addition was finished, the obtained mixture was stirred overnight at room temperature. An inorganic salt was separated by filtration from the reaction mixture to obtain a reaction solution. The inorganic salt was washed with 100 ml of acetone to obtain a washing liquid, and the aforesaid reaction solution and the washing liquid were mixed. After adding 100 ml of water to the obtained solution and removing acetone by reduced-pressure distillation, an extraction process was carried out with 100 ml of ethyl acetate to obtain an organic layer. By concentrating the obtained organic layer, 38.2 g of ethyl piperidin-3-ylcarbamate was obtained as a brown solid, with a yield of 72.1%.

### Reference Example 33: Manufacturing ethyl piperidin-3-ylcarbamate

Into a solution obtained by dissolving 38.2 g (0.23 mol) of ethyl pyridin-3-ylcarbamate obtained in Reference Example 32 in 124 g (2.07 mol) of acetic acid, 13 g of palladium carbon (5%) was added, and the obtained mixture was stirred for 16 hours at a hydrogen pressure of 0.5 MPa and at 65°C. After the reaction was finished, palladium carbon was separated by filtration to obtain a reaction solution. The palladium carbon was washed with 200 ml of water to obtain a washing liquid. The aforesaid reaction solution and the washing liquid were mixed. The obtained solution was added dropwise into a solution obtained in advance by dissolving 92 g (2.30 mol) of sodium hydroxide in 200 ml of water. The inner temperature of the mixture during the dropwise addition was maintained to be 0 to 10°C. After the dropwise addition was finished, an extraction process was carried out with 200 ml of toluene to obtain an organic layer. The water layer was subjected to an extraction process with 200 ml of tetrahydrofuran, and the obtained organic layer was mixed with the aforesaid organic layer. The obtained solution was concentrated under reduced pressure, and the obtained oily substance was dissolved in 150 ml of tetrahydrofuran and dried over anhydrous sodium sulfate. The obtained mixture was subjected to a filtration process, and the obtained solution was subjected to solvent concentration under reduced pressure to obtain 30.9 g of ethyl piperidin-3-ylcarbamate as a brown solid, with a yield of 78.1%.

### Example 34: Manufacturing a diastereomer salt (optical resolution)

A mixture was obtained by mixing 1. 00 g (5.81 mmol) of ethyl piperidin-3-ylcarbamate obtained in Reference Example 33, 0.97 g (6.39 mmol) of R-mandelic acid, and 5 ml of tetrahydrofuran. When the obtained mixture was stirred at room temperature, a homogeneous solution was obtained. When the solution was left to stand quietly at 0 to 5°C for 20 days, crystals were deposited. After the obtained suspension was stirred at room temperature for 5 hours, crystals were collected by filtration. The crystals were washed with 5 ml of tetrahydrofuran, and the obtained crystals were dried to obtain 0.44 g of a diastereomer salt of ethyl (R)-piperidin-3-ylcarbamate and R-mandelic acid as a white crystal, with a yield of 23.3%.
¹H-NMR (DMSO-d₆, 400MHz) δ ppm: 7.32 (2H, d, J=7.8Hz), 7.27-7.08 (4H, m), 4.55 (1H, s), 3.93 (2H, q, J=3.9, 12.2Hz), 3.58-3.46(1H, m), 3.08-2.90 (2H, m), 2.62-2.42 (2H, m), 1.78-1.63 (2H, m), 1.55-1.43 (1H, m), 1.37-1.25 (1H, m), 1.10 (3H, t, J=7.3Hz) ¹³C-NMR (DMSO-d₆, 400MHz) δ ppm: 175.0, 155.3, 143.0, 127.3, 126.2, 126.1, 73.3, 59.7, 47.0, 45.0, 42.8, 28.7, 21.1, 14.6

With use of triethylamine, ethyl piperidin-3-ylcarbamate was taken out from the diastereomer salt, and this was derivatized with use of 3,5-dinitrobenzoyl chloride and analyzed by high-speed liquid chromatography, whereby the optical purity of the ethyl piperidin-3-ylcarbamate in the diastereomer salt was 95.8%ee (R form).

### <optical purity analysis condition>

column: CHIRALCEL AS-RH (4.6 * 150 mm, 5 µm)
mobile phase: A = water, B = acetonitrile, A/B = 65/35
flow rate: 1.0 ml/minute
detector: UV 254 nm
holding time: S form = 8.1 minutes, R form = 16.8 minutes
Example 35: Optical resolution of ethyl piperidin-3-ylcarbamate

A mixture was obtained by mixing 1. 00 g (5. 81 mmol) of ethyl piperidin-3-ylcarbamate obtained in Reference Example 33, 0.97 g (6.39 mmol) of R-mandelic acid, and 5 ml of 2-propanol. When the obtained mixture was stirred at room temperature, a homogeneous solution was obtained. When the solution was left to stand quietly at 0 to 5°C for 21 days, crystals were deposited. After the obtained suspension was stirred overnight at room temperature, crystals were collected by filtration. The crystals were washed with 5 ml of 2-propanol, and the obtained crystals were dried to obtain 0.32 g of a diastereomer salt of ethyl (R)-piperidin-3-ylcarbamate and R-mandelic acid as a white crystal, with a yield of 17.0%.

Analysis was carried out by the same method as in Reference Example 20, with a result that the optical purity of ethyl piperidin-3-ylcarbamate in the diastereomer salt was 91.0%ee (R form).

### Example 36, Comparative Examples 1 to 5: Optical resolution of t-butyl piperidin-3-ylcarbamate

A mixture was obtained by mixing 100 mg (0.5 mmol) of the t-butyl piperidin-3-ylcarbamate obtained by the same method as in Reference Example 27, 0.55 mmol of an optically active acid described in Table 3, and 2 ml of a solvent described in Table 3 at room temperature, so as to manufacture a diastereomer salt of t-butyl piperidin-3-ylcarbamate and the optically active acid. When the diastereomer salt was crystallized, the deposited crystals were collected by filtration, and the diastereomer salt obtained by drying was analyzed by an optical purity analysis method described below. The result is shown in Table 3.

### <optical purity analysis condition>

column: CHIRALCEL (registered trademark) AS-RH (4.6 X 150 mm, 5 µm)
mobile phase: A = water, B = acetonitrile, A/B = 60/40 flow rate: 1.0 ml/minute
detector: UV 254 nm
holding time: S form = 10.4 minutes, R form = 15.6 minutes

**TABLE 3**

| Experiment number | Optically active acid | Result | Ssolvent | | | |
|---|---|---|---|---|---|---|
| | | | Ethyl acetate | 2-propanol | Acetone | Tetrahydrofuran |
| Example 36 | R-mandelic acid | Crystallization Yield | Present 49% | Present 25% | Present 33% | Present 37% |
| | | Optical purity | 66%ee (R) | 92%ee (R) | 88%ee (R) | 92%ee (R) |
| Comparative Example 1 | D-camphorsulfonic acid | Crystallization Yield | Present 71% | Absent | Present 50% | Absent |
| | | Optical purity | 1%ee (R) | | 6%ee (R) | |
| Comparative Example 2 | L-malic acid | Crystallization Yield | Absent | Absent | Absent | Absent |
| | | Optical purity | | | | |
| Comparative Example 3 | L-dibenzoyloxalic acid | Crystallization Yield | Absent | Absent | Absent | Absent |
| | | Optical purity | | | | |
| Comparative Example 4 | L-ditoluoyloxalic acid | Crystallization Yield | Absent | Absent | Absent | Absent |
| | | Optical purity | | | | |
| Comparative Example 5 | D-di-p-anisoyloxalic acid | Crystallization Yield | Present 86% | Absent | Absent | Absent |
| | | Optical purity | 2%ee (R) | | | |

### INDUSTRIAL APPLICABILITY

A piperidin-3-ylcarbamate compound obtained by the present invention is useful, for example, as a synthesis intermediate of a diabetes treating drug (See International Application Publication No. 2005/085246 and International Application Publication No. 2006/112331), and the present invention is industrially applicable as a manufacturing method for such an intermediate.

## Claims

1. An optical resolution method of a
piperidin-3-ylcarbamate compound **characterized in that** an RS mixture of a piperidin-3-ylcarbamate compound represented by the formula (1) : 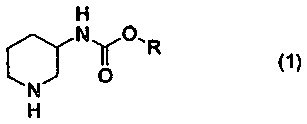 (in the formula, R represents an ethyl group or a t-butyl group) and optically active mandelic acid are brought into contact.

2. A manufacturing method for an optically active piperidin-3-ylcarbamate compound represented by the formula (2) : 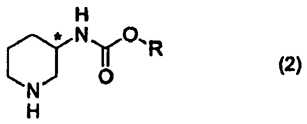 (in the formula, R represents an ethyl group or a t-butyl group, and * represents that the carbon atom is an optically active center) or a salt thereof, comprising the optical resolution method of claim 1 to crystallize a diastereomer salt of the optically active piperidin-3-ylcarbamate compound and the optically active mandelic acid, and then allowing an acid or an alkali to react with the diastereomer salt.

3. The manufacturing method according to claim 2, wherein the optically active mandelic acid is R-mandelic acid, and the obtained optically active piperidin-3-ylcarbamate compound represented by the formula (2) is an R form.

4. A diastereomer salt of optically active ethyl piperidin-3-ylcarbamate and optically active mandelic acid, or a diastereomer salt of optically active t-butyl piperidin-3-ylcarbamate and optically active mandelic acid.

5. The first diastereomer salt of claim 4, wherein the optically active ethyl piperidine-3-ylcarbamate is ethyl (R)-piperidin-3-ylcarbamate and the optically active mandelic acid is R-mandelic acid, or the second diastereomer salt of claim 4, wherein the optically active t-butyl piperidine-3-ylcarbamate is t-butyl (R)-piperidin-3-ylcarbamate and the optically active mandelic acid is R-mandelic acid.

## Patentansprüche

1. Verfahren zur optischen Auflösung einer Piperidin-3-ylcarbamat-Verbindung, **dadurch gekennzeichnet, dass** ein RS-Gemisch aus einer durch die Formel (1) dargestellten Piperidin-3-ylcarbamat-Verbindung 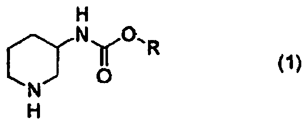 (wobei R in der Formel eine Ethylgruppe oder eine t-Butylgruppe darstellt) und optisch aktive Mandelsäure in Kontakt gebracht werden.

2. Herstellungsverfahren für eine durch die Formel (2) dargestellte optisch aktive Piperidin-3-ylcarbamat-Verbindung 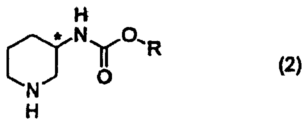 (wobei R in der Formel eine Ethylgruppe oder eine t-Butylgruppe darstellt, und * darstellt, dass das Kohlenstoffatom ein optisch aktives Zentrum ist) oder ein Salz davon, umfassend das Verfahren zur optischen Auflösung nach Anspruch 1 zum Kristallisieren eines Diastereomersalzes der optisch aktiven Piperidin-3-ylcarbamat-Verbindung und der optisch aktiven Mandelsäure, und dann Reagieren lassen einer Säure oder eines Alkalis mit dem Diastereomersalz.

3. Herstellungsverfahren nach Anspruch 2, wobei die optisch aktive Mandelsäure R-Mandelsäure ist, und die erhaltene optisch aktive Piperidin-3-ylcarbamat-Verbindung, dargestellt durch die Formel (2), eine *R*-Form ist.

4. Diastereomersalz von optisch aktivem Ethylpiperidin-3-ylcarbamat und optisch aktiver Mandelsäure, oder ein Diastereomersalz von optisch aktivem t-Butylpiperidin-3-ylcarbamat und optisch aktiver Mandelsäure.

5. Erstes Diastereomer-Salz nach Anspruch 4, wobei das optisch aktive Ethylpiperidin-3-ylcarbamat Ethyl-(*R*)-piperidin-3-ylcarbamat ist und die optisch aktive Mandelsäure *R*-Mandelsäure ist, oder das zweite Diastereomersalz nach Anspruch 4, wobei das optisch aktive t-Butylpiperidin-3-ylcarbamat t-Butyl-(*R*)-piperidin-3-ylcarbamat ist und die optisch aktive Mandelsäure *R*-Mandelsäure ist.

## Revendications

1. Procédé de résolution optique d'un composé de pipéridin-3-ylcarbamate **caractérisé en ce qu'**un mélange RS d'un composé de pipéridin-3-ylcarbamate représenté par la formule (1): 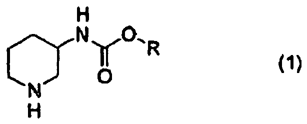 (dans la formule, R représente un groupe éthyle ou un groupe t-butyle) et un acide mandélique optiquement actif sont amenés en contact.

2. Procédé de fabrication pour un composé de pipéridin-3-ylcarbamate optiquement actif représenté par la formule (2): 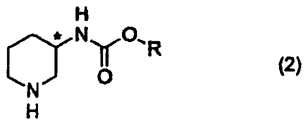 (dans la formule, R représente un groupe éthyle ou un groupe t-butyle et * indique que l'atome de carbone est un centre optiquement actif) ou un sel de celui-ci, comprenant le procédé de résolution optique selon la revendication 1 pour cristalliser un sel diastéréoisomère du composé de pipéridin-3-ylcarbamate optiquement actif et de l'acide mandélique optiquement actif et consistant ensuite à laisser un acide ou un alcalin réagir avec le sel diastéréoisomère.

3. Procédé de fabrication selon la revendication 2, dans lequel l'acide mandélique optiquement actif est l'acide R-mandélique et le composé de pipéridin-3-ylcarbamate optiquement actif obtenu représenté par la formule (2) est une forme R.

4. Sel diastéréoisomère de pipéridin-3-ylcarbamate d'éthyle optiquement actif et d'acide mandélique optiquement actif ou sel diastéréoisomère de pipéridin-3-ylcarbamate de t-butyle optiquement actif et d'acide mandélique optiquement actif

5. Premier sel diastéréoisomère selon la revendication 4, dans lequel le pipéridin-3-ylcarbamate d'éthyle optiquement actif est le (R)-pipéridin-3-ylcarbamate d'éthyle et l'acide mandélique optiquement actif est l'acide R-mandélique ou deuxième sel diastéréoisomère selon la revendication 4, dans lequel le pipéridin-3-ylcarbamate de t-butyle optiquement actif est le (R)-pipéridin-3-ylcarbamate de t-butyle et l'acide mandélique optiquement actif est l'acide R-mandélique.
